# EUROPEAN PATENT SPECIFICATION

(11) **EP 3 149 046 B1**
(45) Date of publication and mention of the grant of the patent: **26.02.2020**
(21) Application number: 15853632.6
(22) Date of filing: 24.07.2015
(51) Int. Cl.: C07K 16/28, C07K 16/46, A61K 39/395, C12N 15/13, C12N 15/63, C12N 5/10, A61P 35/02, A61P 37/00

(54) **HUMANIZED ANTI-CD19 ANTIBODY AND USE THEREOF**
HUMANISIERTER ANTI-CD19-ANTIKÖRPER UND VERWENDUNG DAVON
ANTICORPS HUMANISÉ ANTI-CD19 ET UTILISATION ASSOCIÉE

(43) Date of publication of application: 05.04.2017
(73) Proprietor: Innovative Cellular Therapeutics Co., Ltd., Shanghai 201203 (CN)
(72) Inventor: WU, Zhao, Shanghai 201203 (CN); LIU, Zhigang, Beijing 100071 (CN)
(74) Representative: Abel & Imray
(86) International application number: PCT/CN2015/084991
(87) International publication number: WO 2017/015783

(56) References cited:
- WO-A1-2014/153270
- WO-A2-2005/012493
- WO-A2-2010/095031
- WO-A2-2010/095031
- WO-A2-2010/102276
- WO-A2-2013/138244
- BIPULENDU JENA ET AL: "Chimeric Antigen Receptor (CAR)-Specific Monoclonal Antibody to Detect CD19-Specific T Cells in Clinical Trials", PLOS ONE, vol. 8, no. 3, 1 March 2013 (2013-03-01), page e57838, XP055122892, DOI: 10.1371/journal.pone.0057838
- ZOLA H ET AL: "Preparation and characterization of a chimeric CD19 monoclonal antibody", IMMUNOLOGY AND CELL BIO, CARLTON, AU, vol. 69, no. 6, 1 December 1991 (1991-12-01), pages 411-422, XP008115309, ISSN: 0818-9641, DOI: 10.1038/ICB.1991.58
- KURAMOCHI T ET AL: "Humanization and Simultaneous Optimization of Monoclonal Antibody", ANTIBODY-DRUG CONJUGATES ( BOOK SERIE:METHODS IN MOLECULAR BIOLOGY), HUMANA PRESS, US, vol. 1060, 1 January 2014 (2014-01-01), pages 123-137, XP009176367, ISSN: 1064-3745, DOI: 10.1007/978-1-62703-586-6_7 ISBN: 978-1-62703-541-5
- Olivier Léger ET AL: "Antibody Drug Discovery Chapter 1: "Humanization of Antibodies"" In: "Molecular Medicine and Medicinal Chemistry", 1 January 2011 (2011-01-01), XP055119233, pages 1-23, * the whole document *
- KUGLER M ET AL.: 'Stabilization and humanization of a single-chain Fv antibody fragment specific for human lymphocyte antigen CD 19 by designed point mutations and CDR-grafting onto a human framework' PROTEIN ENG DES SEL vol. 22, no. 3, 31 March 2009, pages 135 - 147, XP002575212

## Description

### TECHNICAL FIELD

This disclosure relates to biotechnology. More specifically, the disclosure relates to humanized antibodies or fragments for binding to CD19 and use thereof.

### BACKGROUND

CD19 is a hallmark of B-cells since CD19 is expressed on the surface of human B cells including pre-B cells, immature B cells, mature B cells and malignant B cells. CD19 has been used to diagnose cancers that arise from B cell (e.g., B-cell lymphomas). Further, some drugs associated with anti-CD19 may direct treatment specifically towards B-cell cancers. For example, CD19 monoclonal antibody may be used for treatment of B-cell related diseases.

Monoclonal antibodies are typically made entirely from mouse cells. One problem with this is that the human immune system may see these antibodies as foreign and may mount a response against them. In the short term, this can sometimes cause an immune response. In the long term. This may cause the antibodies to only work the first time(s) they are given. After the first time, the human immune system may destroy these antibodies before the desired treatment takes place.

WO 2014/153270 (Novartis AG; The Trustees of the University of Pennsylvania) discloses compositions and methods for treating diseases associated with expression of CD19; chimeric antigen receptor (CAR) specific to CD19, vectors encoding the same, and recombinant T cells comprising the CD19 CAR; and methods of administering a genetically modified T cell expressing a CAR that comprises a CD19 binding domain.

### SUMMARY

Embodiments herein relate to a humanized antibody or fragment that binds to human CD19. The humanized antibody or fragment includes a heavy chain variable region (HVR) sequence including the amino acid sequence of SEQ ID NO: 27 and a light chain variable region (LVR) sequence including the amino acid sequences of SEQ ID NO: 34, the amino acid sequences of SEQ ID NO: 35, or the amino acid sequences of SEQ ID NO: 36.

The invention also provides the humanized antibody or fragment for use in therapy, for example for use in the treatment of a CD19 positive tumor or for use in the treatment of an autoimmune disease.

In some embodiments, the HVR sequence may include at least one of a complementarity determining region (CDR) sequence of SEQ ID NO: 7, a CDR sequence of SEQ ID NO: 8, and a CDR sequence of SEQ ID NO: 9, and the HVR sequence may include a framework region (FR) sequence of SEQ ID NO: 24. In certain embodiments, the LVR sequence may include the amino acid sequences of SEQ ID NO: 34. In certain embodiments, the LVR sequence may include the amino acid sequences of SEQ ID NO: 35. In certain embodiments, the LVR sequence may include the amino acid sequences of SEQ ID NO: 36.

In some embodiments, the HVR sequence may include at least one of a complementarity determining region (CDR) sequence of SEQ ID NO: 7, a CDR sequence of SEQ ID NO: 8, and a CDR sequence of SEQ ID NO: 9.

In some embodiments, the HVR sequence may include a framework region (FR) sequence of SEQ ID NO: 24.

In some embodiments, the LVR sequence may include the amino acid sequences of SEQ ID NO: 34.

In some embodiments, the LVR sequence may include the amino acid sequences of SEQ ID NO: 35.

In some embodiments, the LVR sequence may include the amino acid sequences of SEQ ID NO: 36.

In some embodiments, the HVR is joined to a human IgG chain constant region. In these instances, the human IgG may include IgG1 or IgG3.

In some embodiments, the humanized antibody may include a conjugated to a cytotoxic agent. In these instances, the cytotoxic agent may include a radioactive isotope or a toxin.

In some embodiments, the humanized antibody may be conjugated to a sequence derived from 4-1 BB or CD28, or a combination thereof.

In some embodiments, the humanized antibody or fragment may be produced in a HEK293 cell.

In some embodiments, the humanized antibody or fragment may be produced in a HEK293 cell, a B cell, a T cell, a NK cell, an embryonic cell, a dendritic cell or a macrophage.

The embodiments herein further relate to a composition including the humanized antibody or fragment and a pharmaceutically acceptable carrier.

The invention also provides the composition including the humanized antibody or fragment for use in therapy, for example for use in the treatment of a CD19 positive tumor or for use in the treatment of an autoimmune disease.

Also disclosed herein is an article of manufacture including a container and a composition contained therein. The composition may include the humanized antibody.

Also disclosed herein is a polynucleotide that encodes the humanized antibody.

Also disclosed herein is an expression vector encoding the humanized antibody.

Also disclosed herein is a host cell including a nucleic acid encoding the humanized antibody.

The embodiments herein find utility in a method of treating a CD19 positive tumor. The method may include administering to a subject having a CD19 positive tumor a therapeutically effective amount of the humanized antibody.

The embodiments herein find utility in a method of treating an autoimmune disease. The method may include administering to a subject having an autoimmune disease a therapeutically effective amount of the humanized antibody.

The embodiments herein find utility in a method of treating a CD19 related disorder. The method may include administering to a subject having an autoimmune disease a therapeutically effective amount of the humanized antibody or fragment. The CD19 related disorder may include at least one of a B-cell lymphoma, leukemia or an autoimmune disease in a subject.

Also disclosed herein is a modified cell including a chimeric antigen receptor (CAR) that includes an antigen recognition domain and an intracellular domain. The antigen domain may include the humanized antibody.

The embodiments herein find utility in a method for treating a subject having a CD19 positive tumor. Disclosed herein the method may include administering a modified cell to the subject having the CD19 positive tumor. The modified cell may include an intracellular domain and an antigen recognition domain that includes the humanized antibody.

Disclosed herein, the modified cell may include at least one of a B cell, a T cell, a NK cell, an embryonic cell, a dendritic cell or a macrophage.

Disclosed herein, the genetically modified cell replicates in vivo.

Disclosed herein, the modified cell may form memory cells in the subject.

Disclosed herein, the modified cells are administered intravenously to the subject.

Disclosed herein, the modified cells persist in the subject.

Disclosed herein, the modified cell is an autologous T cell.

Disclosed herein, the modified cell produces the humanized antibody and/or an intracellular domain.

### BRIEF DESCRIPTION OF THE DRAWINGS

The detailed description is described with reference to the accompanying figures.

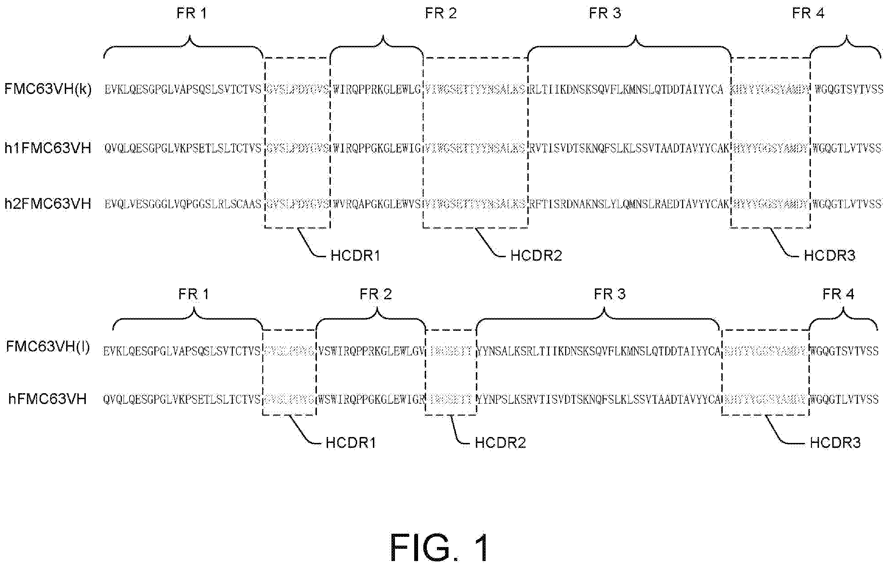

FIG. 1 illustrates a sequence alignment comparing the amino acid sequences of each variable domains of heavy chain of FMC63VH (SEQ ID: 1), hFMC63VH (SEQ ID: 25), h1FMC63VH (SEQ ID: 26), and h2FMC63VH (SEQ ID: 27) according to Kabat or IMGT database definitions.

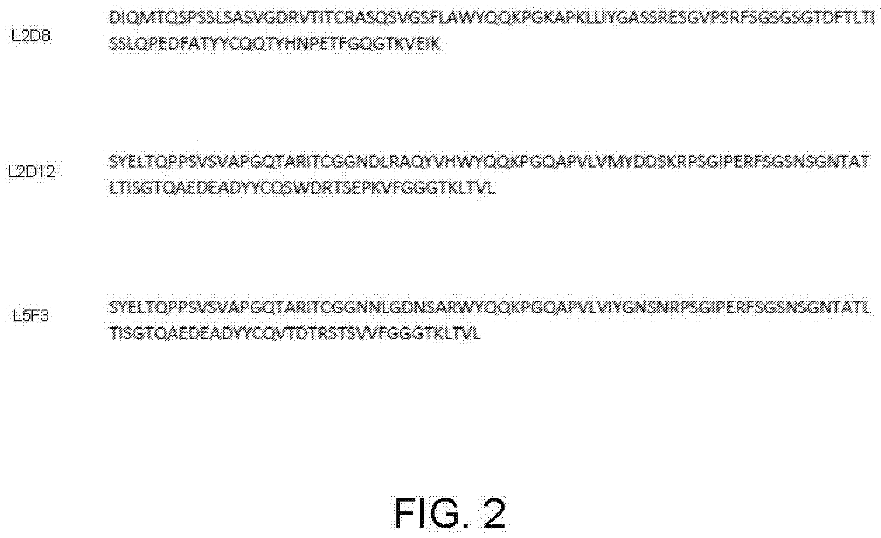

FIG. 2 illustrates a sequence alignment comparing the amino acid sequences of variable domains of light chain of L2D8 (SEQ ID: 34), L2D12 (SEQ ID: 35), and L5F3 (SEQ ID: 36).
FIG. 3 shows the results of reducing SDS-PAGE (10%) analysis of the purified recombinant full length FMC63 antibodies. M indicates protein molecular weight standards (170, 130, 100, 70, 55, 40, 35, 25, 15, 10kD); FMC63 indicates purified recombinant murine monoclonal antibody FMC63. The heavy chain is about 50kD, and the light chain is about 25KD.
FIG. 4 shows the results of reducing SDS-PAGE (15%) analysis of purified recombinant CD19ECD-His fusion protein. M indicates protein molecular weight standards (170, 130, 100, 70, 55, 40, 35, 25, 15, 10kD); "1" indicates purified recombinant CD19ECD-His; "2" indicates purified recombinant CD19ECD-His.
FIG. 5 illustrates results of ELISA analysis of recombinant murine antibody FMC63 in combination with different protein antigens. CD19-His: recombinant human CD19 extracellular domain with the His-tag fusion protein; IL17-His: recombinant human IL17 fusion protein with the His-tag; PCSK9-His: recombinant human PCSK9 with a His-tag fusion protein; BSA: bovine serum albumin protein.
FIG. 6 shows the results of reducing SDS-PAGE (15%) analysis of the purified recombinant four kinds FMC63 full length antibodies. M indicates protein molecular weight standards (170, 130, 100, 70, 55, 40, 35, 25, 15, 10kD); "1" indicates purified recombinant chimeric FMC63; "2" indicates the purified recombinant hFMC63; "3" indicates purified recombinant h2FMC63. The heavy chain is about 50KD, and the light chain is about 25KD.

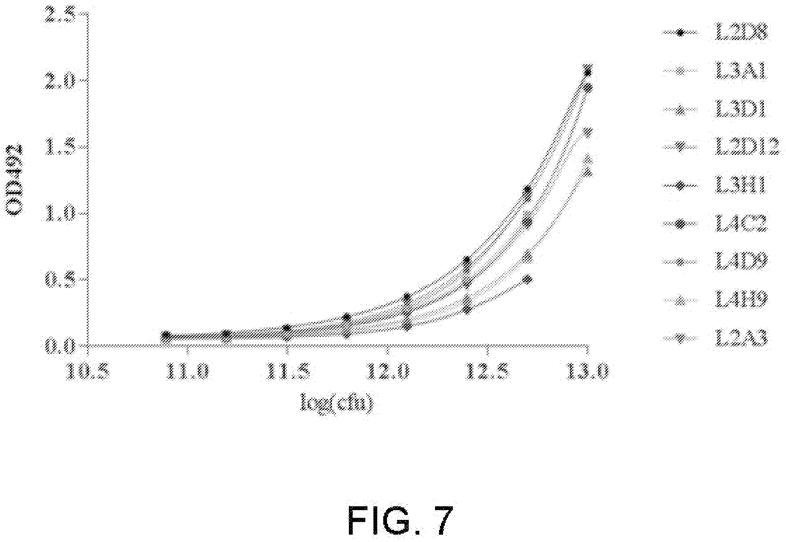

FIG. 7 illustrates comparison between the relative affinities of different monoclonal antibodies measured using purified phage-Fab.

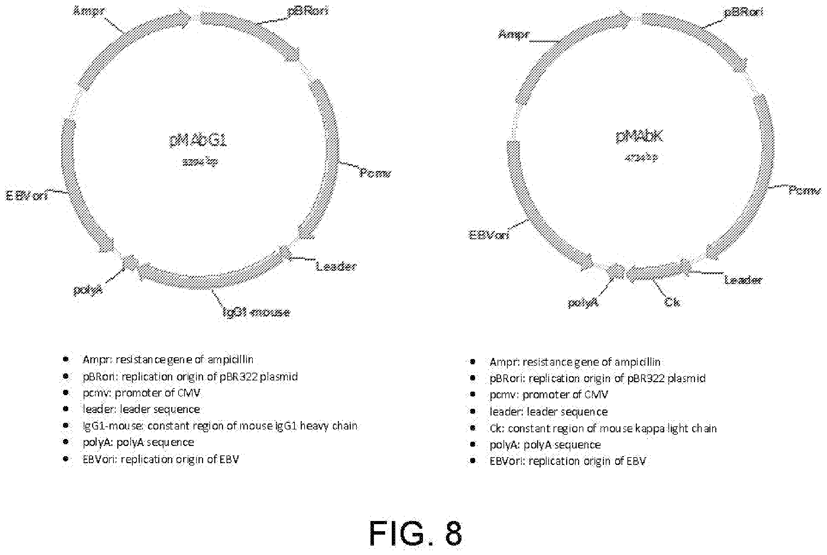

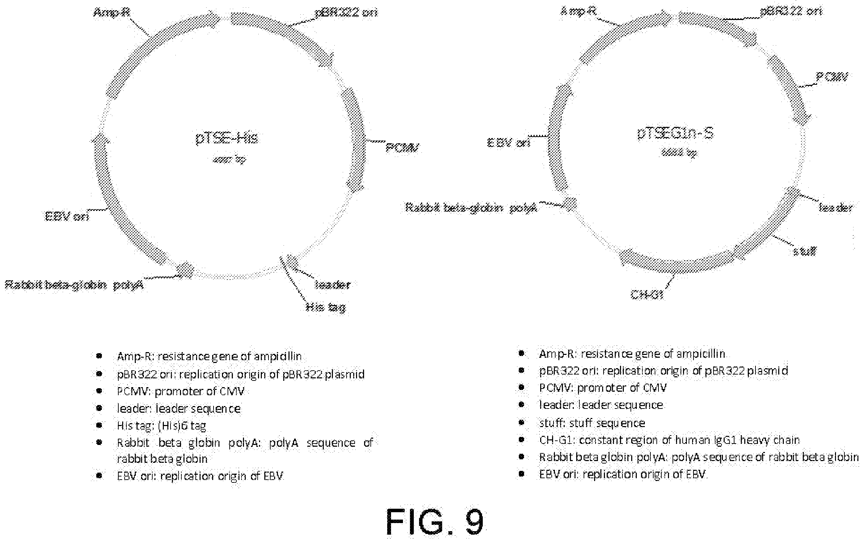

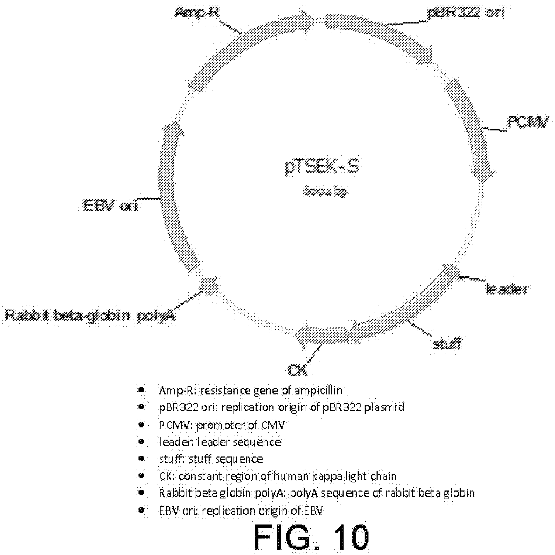

FIGS. 8-10 shows the vectors for expression of one or more polynucleotides in host cells.

### DETAILED DESCRIPTION

Unless defined otherwise, all technical and scientific terms used herein have the same meaning as commonly understood by those of ordinary skill in the art to which the present disclosure belongs. Although any methods and materials similar or equivalent to those described herein can be used in the practice or testing of the present disclosure, methods and materials are described. For the purposes of the present disclosure, the following terms are defined below.

The articles "a" and "an" are used herein to refer to one or to more than one (i.e. to at least one) of the grammatical object of the article. By way of example, "an element" means one element or more than one element.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

By "coding sequence" is meant any nucleic acid sequence that contributes to the code for the polypeptide product of a gene. By contrast, the term "non-coding sequence" refers to any nucleic acid sequence that does not contribute to the code for the polypeptide product of a gene.

Throughout this specification, unless the context requires otherwise, the words "comprise", "comprises" and "comprising" will be understood to imply the inclusion of a stated step or element or group of steps or elements but not the exclusion of any other step or element or group of steps or elements.

By "consisting of" is meant including, and limited to, whatever follows the phrase "consisting of." Thus, the phrase "consisting of" indicates that the listed elements are required or mandatory, and that no other elements may be present.

By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

The terms "complementary" and "complementarity" refer to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands.

By "corresponds to" or "corresponding to" is meant (a) a polynucleotide having a nucleotide sequence that is substantially identical or complementary to all or a portion of a reference polynucleotide sequence or encoding an amino acid sequence identical to an amino acid sequence in a peptide or protein; or (b) a peptide or polypeptide having an amino acid sequence that is substantially identical to a sequence of amino acids in a reference peptide or protein.

As used herein, the terms "function" and "functional" and the like refer to a biological, binding, or therapeutic function.

By "gene" is meant a unit of inheritance that occupies a specific locus on a chromosome and consists of transcriptional and/or translational regulatory sequences and/or a coding region and/or non-translated sequences (i.e., introns, 5' and 3' untranslated sequences).

"Homology" refers to the percentage number of amino acids that are identical or constitute conservative substitutions. Homology may be determined using sequence comparison programs such as GAP (Deveraux et al., 1984, Nucleic Acids Research 12, 387-395).
In this way sequences of a similar or substantially different length to those cited herein could be compared by insertion of gaps into the alignment, such gaps being determined, for example, by the comparison algorithm used by GAP.

The term "host cell" includes an individual cell or cell culture which can be or has been a recipient of any recombinant vector(s) or isolated polynucleotide of the present disclosure. Host cells include progeny of a single host cell, and the progeny may not necessarily be completely identical (in morphology or in total DNA complement) to the original parent cell due to natural, accidental, or deliberate mutation and/or change. A host cell includes cells transfected or infected in vivo or in vitro with a recombinant vector or a polynucleotide of the present disclosure. A host cell which comprises a recombinant vector of the present disclosure is a recombinant host cell. For example, a cell that produces a humanized CD19 antibody of the present disclosure will include the bacterial and eukaryotic host cells into which nucleic acid encoding the antibodies have been introduced.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes.

An "isolated" nucleic acid molecule is a nucleic acid molecule that is identified and separated from at least one contaminant nucleic acid molecule with which it is ordinarily associated in the natural source of the antibody nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

The expression "control sequences" refers to DNA sequences necessary for the expression of an operably linked coding sequence in a particular host organism. The control sequences that are suiTable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The recitation "polynucleotide" or "nucleic acid" as used herein designates mRNA, RNA, cRNA, rRNA, cDNA or DNA. The term typically refers to polymeric form of nucleotides of at least 10 bases in length, either ribonucleotides or deoxynucleotides or a modified form of either type of nucleotide. The term includes single and double stranded forms of DNA and RNA.

The terms "polynucleotide variant" and "variant" and the like refer to polynucleotides displaying substantial sequence identity with a reference polynucleotide sequence or polynucleotides that hybridize with a reference sequence under stringent conditions that are defined hereinafter. These terms also encompass polynucleotides that are distinguished from a reference polynucleotide by the addition, deletion or substitution of at least one nucleotide. Accordingly, the terms "polynucleotide variant" and "variant" include polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains the biological function or activity of the reference polynucleotide, or has increased activity in relation to the reference polynucleotide (i.e., optimized). Polynucleotide variants include, for example, polynucleotides having at least 50% (and at least 51% to at least 99% and all integer percentages in between, e.g., 90%, 95%, or 98%) sequence identity with a reference polynucleotide sequence described herein. The terms "polynucleotide variant" and "variant" also include naturally-occurring allelic variants and orthologs that encode these enzymes.

"Polypeptide," "polypeptide fragment," "peptide" and "protein" are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers. In certain aspects, polypeptides may include enzymatic polypeptides, or "enzymes", which typically catalyze (i.e., increase the rate of) various chemical reactions.

The recitation polypeptide "variant" refers to polypeptides that are distinguished from a reference polypeptide sequence by the addition, deletion or substitution of at least one amino acid residue. In certain embodiments, a polypeptide variant is distinguished from a reference polypeptide by one or more substitutions, which may be conservative or non-conservative. In certain embodiments, the polypeptide variant comprises conservative substitutions and, in this regard, it is well understood in the art that some amino acids may be changed to others with broadly similar properties without changing the nature of the activity of the polypeptide. Polypeptide variants also encompass polypeptides in which one or more amino acids have been added or deleted, or replaced with different amino acid residues.

The term "reference sequence" refers generally to a nucleic acid coding sequence, or amino acid sequence, to which another sequence is being compared. All polypeptide and polynucleotide sequences described herein are included as references sequences.

The recitations "sequence identity" or, for example, comprising a "sequence 50% identical to," as used herein, refer to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. Included are nucleotides and polypeptides having at least about 50%, 55%, 60%, 65%, 70%, 75%, 80%, 85%, 90%, 95%, 97%, 98%, 99% or 100% sequence identity to any of the reference sequences described herein (see, e.g., Sequence Listing), typically where the polypeptide variant maintains at least one biological activity of the reference polypeptide.

By "statistically significant," it is meant that the result was unlikely to have occurred by chance. Statistical significance can be determined by any method known in the art. Commonly used measures of significance include the p-value, which is the frequency or probability with which the observed event would occur, if the null hypothesis were true. If the obtained p-value is smaller than the significance level, then the null hypothesis is rejected. In simple cases, the significance level is defined at a p-value of 0.05 or less.

"Substantially" or "essentially" means nearly totally or completely, for instance, 95%, 96%, 97%, 98%, 99% or greater of some given quantity.

"Treating" or "treatment" or "alleviation" refers to both therapeutic treatment and prophylactic or preventative measures, wherein the object is to prevent or slow down (lessen) the targeted pathologic condition or disorder. For example, a subject is successfully "treated" for a CD19 positive cancer or an autoimmune disease if, after receiving a therapeutic amount of a CD19 antibody or other compositions associated with CD19 antibody of the present disclosure according to the methods of the present disclosure, the subject shows observable and/or measurable reduction in or absence of one or more signs and symptoms of the particular disease. For example, for cancer, reduction in the number of cancer cells or absence of the cancer cells; reduction in the tumor size; inhibition (i.e., slow to some extent and preferably stop) of tumor metastasis; inhibition, to some extent, of tumor growth; increase in length of remission, and/or relief to some extent, one or more of the symptoms associated with the specific cancer; reduced morbidity and mortality, and improvement in quality of life issues. Reduction of the signs or symptoms of a disease may also be felt by the patient. Treatment can achieve a complete response, defined as disappearance of all signs of cancer, or a partial response, wherein the size of the tumor is decreased, preferably by more than 50 percent, more preferably by 75%. A patient is also considered treated if the patient experiences stable disease. In some embodiment, the cancer patients are still progression-free in the cancer after one year, preferably after 15 months. These parameters for assessing successful treatment and improvement in the disease are readily measurable by routine procedures familiar to a physician of appropriate skill in the art.

The terms "modulating" and "altering" include "increasing" and "enhancing" as well as "decreasing" or "reducing," typically in a statistically significant or a physiologically significant amount or degree relative to a control. In specific embodiments, immunological rejection associated with transplantation of the blood substitutes is decreased relative to an unmodified or differently modified stem cell by at least 10%, at least 15%, at least 20%, at least 25%, at least 30%, at least 40%, at least 50%, at least 60%, at least 70%, at least 80%, at least 90%, at least 100%, at least 150%, at least 200%, at least 300%, at least 400%, at least 500%, or at least 1000%.

An "increased" or "enhanced" amount is typically a "statistically significant" amount, and may include an increase that is 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 1.5, 1.6, 1.7. 1.8, etc.) an amount or level described herein.

A "decreased" or "reduced" or "lesser" amount is typically a "statistically significant" amount, and may include a decrease that is about 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 1.5, 1.6, 1.7. 1.8, etc.) an amount or level described herein.

By "obtained from" is meant that a sample such as, for example, a polynucleotide or polypeptide is isolated from, or derived from, a particular source, such as a desired organism or a specific tissue within a desired organism. "Obtained from" can also refer to the situation in which a polynucleotide or polypeptide sequence is isolated from, or derived from, a particular organism or tissue within an organism. For example, a polynucleotide sequence encoding a reference polypeptide described herein may be isolated from a variety of prokaryotic or eukaryotic organisms, or from particular tissues or cells within certain eukaryotic organism. A "therapeutically effective amount" refers to an amount of an antibody or a drug effective to "treat" a disease or disorder in a subject. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. See preceding definition of "treating".

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Vector" includes shuttle and expression vectors. Typically, the plasmid construct will also include an origin of replication (e.g., the ColE1 origin of replication) and a selectable marker (e.g., ampicillin or tetracycline resistance), for replication and selection, respectively, of the plasmids in bacteria. An "expression vector" refers to a vector that contains the necessary control sequences or regulatory elements for expression of the antibodies including antibody fragment of the present disclosure, in bacterial or eukaryotic cells. Suitable vectors are disclosed below.

Embodiments herein relate to humanized antibodies and the user thereof. The embodiments further relate to a humanized antibody that binds to human CD19.

The CD19 antigen is an antigen of about 90 kDa identified, for example, by the HD237 or B4 antibody (Kiesel et al., Leukemia Research II, 12:1119 (1987)). CD19 is found on cells throughout differentiation of B-lineage cells from the stem cell stage through terminal differentiation into plasma cells, including but not limited to, pre-B cells, B cells (including naïve B cells, antigen-stimulated B cells, memory B cells, plasma cells, and B lymphocytes) and follicular dendritic cells. CD19 is also found on B cells in human fetal tissue. In some embodiments, the CD19 antigen targeted by the antibodies of the present disclosure is the human CD19 antigen.

The term "antibody" is used in the broadest sense and specifically covers monoclonal antibodies (including full length monoclonal antibodies), multi-specific antibodies (e.g., bispecific antibodies), and antibody fragments so long as they exhibit the desired biological activity or function.

The biological activity of the CD19 binding and humanized CD19 binding antibodies of the present disclosure will include at least binding of the antibody to human CD19, and/or binding to human and other primate CD19 (including cynomolgus monkey, rhesus monkey, chimpanzees). Typically, the C19 binding agent binds with an affinity of at least about 1×10-7 M, and preferably 10-8 M to 10-9 M, 10-10 M, 10-11 M, or 10-12 M and binds to the predetermined antigen with an affinity that is at least two-fold greater than its affinity for binding to a non-specific antigen (e.g., BSA, casein) other than the predetermined antigen or a closely-related antigen.

"Antibody fragments" comprise a portion of a full length antibody, generally the antigen binding or variable region of the antibody. Examples of antibody fragments include Fab, Fab', F(ab')2, and Fv fragments; diabodies; linear antibodies; single-chain antibody molecules; and multi-specific antibodies formed from antibody fragments.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three complementarity determining regions (CDRs) specific for an antigen) has the ability to recognize and bind antigen, although at a lower affinity than the entire binding site.

Monoclonal antibody as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al., Nature 256:495 (1975), or may be made by recombinant DNA methods (see, e.g., U.S. Pat. No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

Functional fragments of the CD19 binding antibodies of the present disclosure are those fragments that retain binding to CD19 with substantially the same affinity as the intact full length molecule from which they are derived and show biological activity including depleting B cells as measured by in vitro or in vivo assays such as those described herein.

Variable refers to the fact that certain segments of the variable domains (V domains) differ extensively in sequence among antibodies. The V domain mediates antigen binding and defines specificity of a particular antibody for its particular antigen. However, the variability is not evenly distributed across the 10-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991)). The constant domains are not involved directly in binding an antibody to an antigen, but exhibit various effector functions, such as participation of the antibody in antibody dependent cellular cytotoxicity (ADCC).

Hypervariable region refers to the amino acid residues of an antibody which are responsible for antigen-binding. The hypervariable region generally comprises amino acid residues from a CDR (e.g. around about residues 24-34 (L1), 50-56 (L2) and 89-97 (L3) in the VL, and around about 31-35B (H1), 50-65 (H2) and 95-102 (H3) in the VH (Kabat et al., Sequences of Proteins of Immunological Interest, 5th Ed. Public Health Service, National Institutes of Health, Bethesda, Md. (1991)) and/or those residues from a "hypervariable loop" (e.g. residues 26-32 (L1), 50-52 (L2) and 91-96 (L3) in the VL, and 26-32 (H1), 52A-55 (H2) and 96-101 (H3) in the VH (Chothia and Lesk J. Mol. Biol. 196:901-917 (1987)).

Chimeric antibodies (immunoglobulins) have a portion of the heavy and/or light chain identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al. Proc. Natl Acad. Sci. USA 81:6851-6855 (1984)). Humanized antibody as used herein is a subset of chimeric antibodies.

Humanized forms of non-human (e.g., murine) antibodies are chimeric antibodies which contain minimal sequence derived from non-human immunoglobulin. In some embodiments, humanized antibodies are human immunoglobulins (recipient or acceptor antibody) in which hypervariable region residues of the recipient are replaced by hypervariable region residues from a non-human species (donor antibody) such as mouse, rat, rabbit or nonhuman primate having the desired specificity, affinity, and capacity. In some embodiments, humanized antibodies are antibodies derived from human cells or from transgenic animals (typically mice) with express human antibody genes.

Also disclosed herein, the humanized antibody may include a heavy chain variable region (HVR) sequence comprising one of the amino acid sequences of SEQ ID NO: 25, SEQ ID NO: 26, or 27, and the humanized antibody may include a light chain variable region (LVR) sequence including one of the amino acid sequences of SEQ ID NO: 34, SEQ ID NO: 35, or SEQ ID NO: 36.

In embodiments of the invention, the HVR sequence includes the amino acid sequence of SEQ ID NO: 27. In these instances, the HVR sequence may include at least one of a complementarity determining region (CDR) sequence of SEQ ID NO: 7, a CDR sequence of SEQ ID NO: 8, and a CDR sequence of SEQ ID NO: 9.

In some embodiments, the HVR sequence may include a framework region (FR) sequence of SEQ ID NO: 24. In these instances, the LVR sequence may include at least one of the amino acid sequences of SEQ ID NO: 34, the amino acid sequences of SEQ ID NO: 35, or the amino acid sequences of SEQ ID NO: 36.

In some embodiments, the HVR is joined to a human IgG chain constant region. In these instances, the human IgG is IgG1 or IgG3.

In some embodiments, the humanized antibody may include a conjugated to a cytotoxic agent. In these instances, the cytotoxic agent may include a radioactive isotope or a toxin.

Cytotoxic agent may include a substance that inhibits or prevents the function of cells and/or causes destruction of cells. The term is intended to include radioactive isotopes (e.g., 1131, 1125, Y90 and Re186), chemotherapeutic agents, and toxins such as enzymatically active toxins of bacterial, fungal, plant or animal origin, or fragments thereof.

In some embodiments, the humanized antibody is conjugated to a sequence derived from 4-1 BB or CD28, or a combination thereof. The humanized antibody or fragment may be produced in a HEK293 cell, a B cell, a T cell, a NK cell, an embryonic cell, a dendritic cell or a macrophage.

The embodiments herein further relate to one or more compositions that include the humanized antibody. The one or more compositions may further include a pharmaceutically acceptable carrier and/or a container containing the humanized antibody.

Also disclosed herein is a polynucleotide that encodes the humanized antibody, an expression vector encoding the humanized antibody, and/or a host cell containing a nucleic acid encoding the humanized antibody.

The embodiments herein find utility in a method of treating a CD19 related disease. The method includes administering to a subject having the CD19 disease a therapeutically effective amount of the humanized antibody. The CD19 disease may include at least one of CD19 positive tumors, a B-cell lymphoma or leukemia, and/or an autoimmune disease in the subject.

B cell depletion refers to a reduction in B cell levels in an animal or human after drug or antibody treatment, as compared to the B cell level before treatment. B cell levels are measurable using well known assays such as those described in the Experimental Examples. B cell depletion can be complete or partial. In one embodiment, the depletion of CD19 expressing B cells is at least 25%. Not to be limited by any one mechanism, possible mechanisms of B-cell depletion include ADCC, CDC, apoptosis, modulation of calcium flux, or a combination of two or more of the preceding.

An autoimmune disease may refer to a non-malignant disease or disorder arising from and directed against an individual's own (self) antigens and/or tissues.

The embodiments herein find utility in a method for treating a subject having a CD19 positive tumor. Disclosed herein the method includes administering a modified cell to the subject having the CD19 positive tumor. Disclosed herein, the modified cell may produce and/or contain an antigen recognition domain having the humanized antibody and an intracellular domain. In these instances, the modified cell may include at least one of a B cell, a T cell, a NK cell, an embryonic cell, a dendritic cell or a macrophage. These genetically modified cell may obtained from memory cells in the subject and replicates in vivo. The modified cells are administered intravenously to the subject and persist in the subject. Disclosed herein, the modified cell is an autologous T cell.

### Examples

### Recombinant human CD19 antigen and anti-CD19 murine monoclonal antibody

Recombinant anti-human CD19 murine antibody (FMC63) was prepared. Genes of variable domains of heavy chain (SEQ ID NO: 1) and variable domains of light chains (SEQ ID NO: 2) were obtained and further optimized to obtain various polynucleotides encoding FMC63. Gene optimization includes eliminations of E.coli rare codons and eliminations of restriction sites used by related eukaryotic expression systems and phage display systems. Accordingly, recombinant polynucleotides (*FMC63VH*) encoding heavy chains of FMC63 (FMC63VH) and recombinant polynucleotides (*FMC63VK*) encoding light chains of FMC63 (FMC63VK) were designed and synthesized (*See* Table 1).

**Table 1**

| Name | Sequence | SEQ ID NO |
|---|---|---|
| FMC63VH | EVKLQESGPGLVAPSQSLSVTCTVSGVSLPDYGVS WIRQPPRKGLEWLGVIWGSETTYYNSALKSRLTIIKD NSKSQVFLKMNSLQTDDTAIYYCAKHYYYGGSYAM DYWGQGTSVTVSS | 3 |
| FMC63VK | DIQMTQTTSSLSASLGDRVTISCRASQDISKYLNWY QQKPDGTVKLLIYHTSRLHSGVPSRFSGSGSGTDYS LTISNLEQEDIATYFCQQGNTLPYTFGGGTKLEIK | 4 |
| *FMC63VH* | GAGGTGAAACTGCAGGAGTCAGGACCTGGCCTG GTGGCGCCATCACAGAGCCTGTCCGTCACATGCA CTGTCTCAGGGGTCTCATTACCTGACTATGGTGTA AGCTGGATTCGCCAGCCTCCACGCAAGGGTCTGG AGTGGCTGGGAGTAATCTGGGGTAGTGAAACCAC ATACTATAATTCAGCTCTCAAATCCCGCCTGACCA TCATCAAGGACAACTCCAAGAGCCAAGTTTTCTTA AAAATGAACAGTCTGCAAACTGATGACACAGCCAT TTACTACTGTGCCAAACATTATTACTACGGTGGTA GCTATGCTATGGACTACTGGGGTCAAGGAACCTC AGTCACCGTCTCCTCA | 5 |
| *FMC63VK* | GACATCCAGATGACACAGACTACATCCTCCCTGTC TGCCTCTCTGGGAGACCGCGTCACCATCAGTTGC CGTGCAAGTCAGGACATTAGTAAATACTTAAATTG GTATCAGCAGAAACCAGATGGAACTGTTAAACTCC TGATCTACCATACATCACGCTTACACTCAGGAGTC CCATCACGGTTCAGTGGCAGTGGGTCTGGAACAG ATTATTCTCTCACCATTAGCAACCTGGAGCAAGAA GATATTGCCACTTACTTTTGCCAACAGGGTAATAC GCTTCCGTACACGTTCGGAGGGGGGACTAAGTTG GAAATCAAA | 6 |

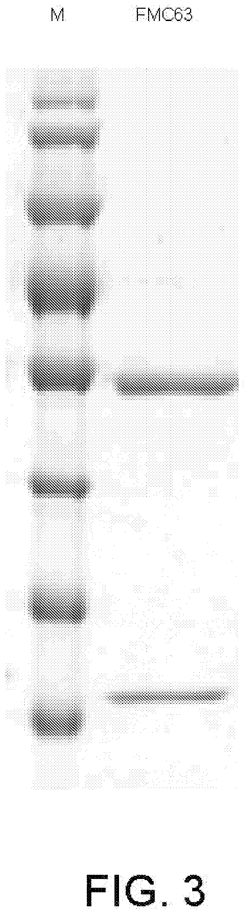

The synthesized polynucleotides encoding FMC63VH and the synthesized polynucleotides FMC63VK were cloned to two expression vectors: pMABG1 and pMABK, respectively (see tu). The plasmids expressing each of the purified heavy and light chains were co-transfected HEK293 cells. Recombinant FMC63 was expressed and obtained by using GE's Protein G affinity chromatography. Electrophoresis indicates that the purity of FMC63 is greater than 95% (FIG. 3).

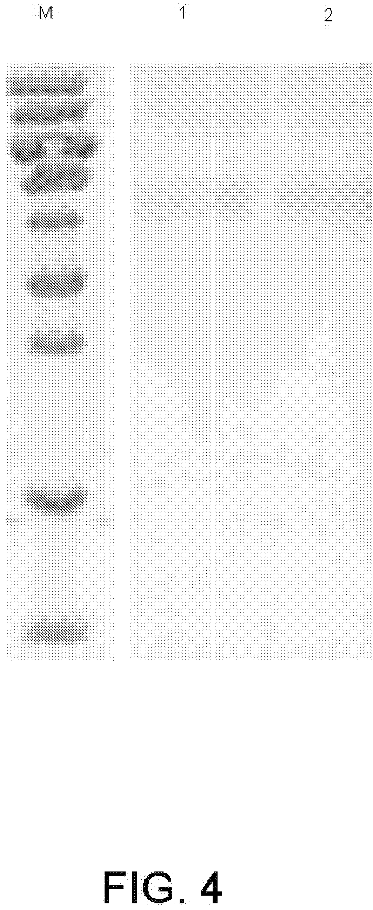

Recombinant human CD19 extracellular domain (CD19-ECD) were prepared. Polynucleotides encoding the CD19 extracellular domain was cloned into expression vector pTSE-His to construct recombinant CD19 antigen. The purified plasmids were transfected into HEK293 cells and the expressed recombinant extracellular domain of CD19 (CD19-ECD-His) was purified using GE's Histrap FF affinity chromatography. SDS-PAGE shows a molecular weight of about 45-50KD due to five glycosylation sites that CD19-ECD has and due to glycosylation heterogeneity when CD19-ECD was expressed in HEK293 cells (FIG. 4).

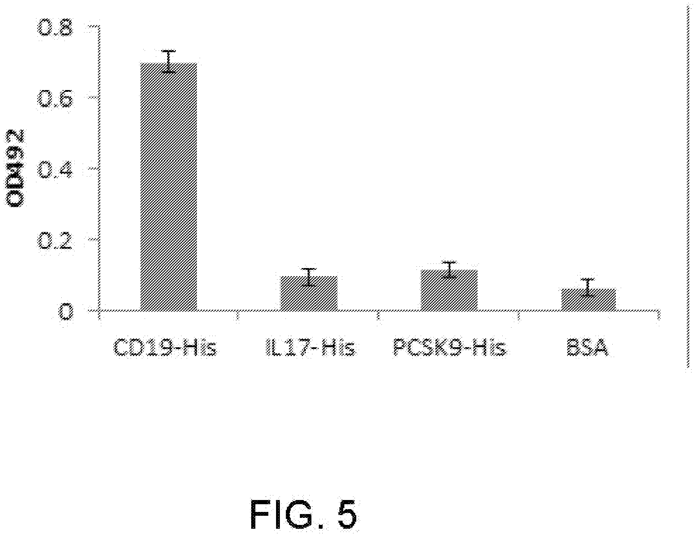

Recombinant murine antibody FMC63 features were verified. The binding capacity between the recombinant FMC63 and recombinant human CD19 antigen was detected using ELISA. The results shows that the recombinant murine antibody is capable of specifically binding to recombinant human CD19-ECD (FIG. 5).

### Humanization of heavy chains of murine antibody FMC63

Humanized FMC63 heavy chain variable region was designed. First, the heavy chain complementarity-determining regions (HCDRs) and framework regions (FRs) of FMC63VH were determined. According to the definition of Kabat database, sequences of the HCDRs and FRs of FMC63VH are provided in Table 2.

**Table 2**

| Name | Sequence | SEQ ID NO: |
|---|---|---|
| FMC63VH | EVKLQESGPGLVAPSQSLSVTCTVSGVSLPDY GVSWIRQPPRKGLEWLGVIWGSETTYYNSALK SRLTIIKDNSKSQVFLKMNSLQTDDTAIYYCAKH YYYGGSYAMDYWGQGTSVTVSS | 3 |
| HCDR1-Kabat | GVSLPDYGVS | 7 |
| HCDR2-Kabat | VIWGSETTYYNSALKS | 8 |
| HCDR3-Kabat | KHYYYGGSYAMDY | 9 |
| FR1- Kabat | EVKLQESGPGLVAPSQSLSVTCTVS | 10 |
| FR2- Kabat | WIRQPPRKGLEWLG | 11 |
| FR3- Kabat | RLTIIKDNSKSQVFLKMNSLQTDDTAIYYCA | 12 |
| FR3- Kabat | WGQGTSVTVSS | 13 |

According to IMGT database definitions, HCDRs and FRs of FMC63VH are provided in Table 3.

**Table 3**

| Name | Sequence | SEQ ID NO: |
|---|---|---|
| FMC63VH | EVKLQESGPGLVAPSQSLSVTCTVSGVSLPD YGVSWIRQPPRKGLEWLGVIWGSETTYYNSA LKSRLTIIKDNSKSQVFLKMNSLQTDDTAIYYC AKHYYYGGSYAMDYWGQGTSVTVSS | 3 |
| HCDR1-IMGT | GVSLPDYG | 14 |
| HCDR2-IMGT | IWGSETT | 15 |
| HCDR3-IMGT | KHYYYGGSYAMDY | 16 |
| FR1-IMGT | EVKLQESGPGLVAPSQSLSVTCTVS | 17 |
| FR2-IMGT | VSWIRQPPRKGLEWLGV | 18 |
| FR3-IMGT | YYNSALKSRLTIIKDNSKSQVFLKMNSLQTDD TAIYYCA | 19 |
| FR3 | WGQGTSVTVSS | 20 |

Humanized templates were selected, and searches were performed using NCBI / IGBLAST tool to search for the highest homology to human antibody genes template IGHV4-59 * 05 based on FR1-CDR1-FR2-CDR2-FR3 sequence of FMC63VH. Searches were also performed using NCBI / IGBLAST tool to highest homology search of human antibody genes template IGHV3-48 * 03 based on FR1-FR2-FR3 sequence of FMC63VH. Searches were also performed using NCBI / IGBLAST tool to search for homology island's tallest man J region sequence IGHJ4 * 01 and IGHJ6 * 01 based on FR4 sequence of FMC63VH. Sequences of the templates are provided in Table 4.

**Table 4**

| Name | Sequence | SEQ ID NO: |
|---|---|---|
| U03893\|IGHV3-48*03 | EVQLVESGGGLVQPGGSLRLSCAASGFT FSSYEMNWVRQAPGKGLEWVSYISSSGS TIYYADSVKGRFTISRDNAKNSLYLQMNSL RAEDTAVYYCAR | 21 |
| M95118\|IGHV4-59*05 | QVQLQESGPGLVKPSETLSLTCTVSGGSI SSYYWSWIRQPPGKGLEWIGRIYYSGSTY YNPSLKSRVTISVDTSKNQFSLKLSSVTAA DTAVYYCA | 22 |
| J00256\|IGHJ6*0 1 | YYYYYGMDVWGQGTTVTVSS | 23 |
| J00256\|IGHJ4*0 1\|Homo sapiens\|F | YFDYWGQGTLVTVSS | 24 |

Humanized HCDRs of FMC63 were designed. Based CDR grafting strategy, various templates were selected, and three different humanized FMC63VH molecules were designed accordingly. Comparison between three different humanized FMC63VH are summarized in Table 5.

**TABLE 5**

| Polynucleotide Names | HCDR definitions | FR1-FR3 template | FR4 template | SEQ ID NO: |
|---|---|---|---|---|
| hFMC63VH | IMGT | IGHV4-59*05 | IGHJ4*01 | 25 |
| h1FMC63VH | Kabat | IGHV4-59*05 | IGHJ4*01 | 26 |
| h2FMC63VH | Kabat | IGHV3-48*03 | IGHJ4*01 | 27 |

With respect to *hFMC63VH,* the HCDRs of FMC63VH were transplanted to selected frame areas of IGHV4-59 * 05. The definition of HCDR is in accordance with the reference IMGT database, and IGHJ4 * 01 was select for FR4. The first version polynucleotides encoding the humanized heavy chain hFMC63VH was obtained accordingly. With respect to *h1FMC63VH,* the HCDRs of FMC63VH were transplanted to select the frame area IGHV4-59 * 05. The definition of HCDR is in accordance with the reference kabat database, and IGHJ4 * 01 was selected for FR4. The second version polynucleotides encoding humanized heavy chain *h1FMC63VH* was obtained accordingly. With respect to h2FMC63VH, the HCDRs of FMC63VH were transplanted to choose IGHV3-48 * 03 framework regions. The definition of HCDR is in accordance with the reference kabat HCDR database, and IGHJ6 * 01 was selected for FR4. The third version polynucleotides encoding the humanized FMC63VH polynucleotide h1FMC63VH was obtained accordingly.

Humanized FMC63VH were cloned and then expressed. Synthesized polynucleotides of three humanized heavy chain variable regions (hFMC63VH, h1FMC63VH, h2FMC63VH) are provided in Table 6.

**TABLE 6**

| Polynucleotides | Sequences | SEQ ID NO: |
|---|---|---|
| *hFMC63VH* | CAGGTGCAACTGCAGGAGTCGGGCCCAGG ACTGGTGAAGCCTTCGGAGACCCTGTCCCT CACCTGCACTGTCTCTGGGGTCTCATTACC AGACTATGGTTGGAGCTGGATTCGGCAGC CGCCGGGGAAGGGACTGGAGTGGATTGGG CGTATCTGGGGTAGTGAAACCACATACTAC AACCCGTCCCTCAAGAGTCGCGTCACCATT TCCGTAGACACGTCCAAGAACCAGTTCTCC CTGAAGCTGAGCTCTGTGACCGCCGCAGA CACGGCTGTGTATTACTGTGCGAAACATTA TTACTACGGTGGTAGCTATGCTATGGACTA CTGGGGCCAAGGAACCCTGGTCACCGTCT CCTCA | 28 |
| *h1FMC63VH* | CAGGTGCAACTGCAGGAGTCGGGCCCAGG ACTGGTGAAGCCTTCGGAGACCCTGTCCCT CACCTGCACTGTCTCTGGGGTCTCATTACC AGACTATGGTGTGAGCTGGATTCGGCAGC CGCCGGGGAAGGGACTGGAGTGGATTGGG GTTATCTGGGGTAGTGAAACCACATACTAC AACAGCGCCCTCAAGAGTCGCGTCACCATT | 29 |
| | TCCGTAGACACGTCCAAGAACCAGTTCTCC CTGAAGCTGAGCTCTGTGACCGCCGCAGA CACGGCTGTGTATTACTGTGCGAAACATTA TTACTACGGTGGTAGCTATGCTATGGACTA CTGGGGCCAAGGAACCCTGGTCACCGTCT CCTCA | |
| *h2FMC63VH* | GAGGTGCAGCTGGTGGAGTCTGGGGGAGG CTTGGTACAGCCTGGGGGGTCCCTGAGAC TCTCCTGTGCAGCCTCTGGAGTGTCCCTGC CTGATTATGGCGTGTCCTGGGTCCGCCAG GCTCCAGGGAAGGGGCTGGAGTGGGTTTC AGTGATCTGGGGCAGCGAGACAACCTACTA CAACAGCGCCCTGAAGTCCCGATTCACCAT CTCCAGAGACAATGCCAAGAACTCACTGTA TCTGCAAATGAACAGCCTGAGAGCCGAGG ACACGGCTGTGTATTACTGTGCGAAGCACT ACTACTACGGCGGCAGCTACGCTATGGACT ACTGGGGCCAAGGAACCCTGGTCACCGTC TCCTCA | 30 |

Then these three humanized antibody heavy chain polynucleotides were expressed in vectors: pTSEG1n-S; named pTSEG1n-hFMC63VH, pTSEG1n-h1 FMC63VH, and pTSEG1n-h2FMC63VH. The heavy chain gene encoding FMC63VH was also cloned to the expression vector pTSEG1n-S including the human antibody heavy chain gene. Accordingly, the expression vector (pTSEG1n-FMC63VH) was constructed to contain the murine-human chimeric heavy chain.

The light chain genes FMC63VK of murine donor was clone to the expression vector pTSEK-S containing the human antibody light chain. Accordingly, the expression vector (pTSEK-FMC63VK) was constructed to contain the murine-human chimeric light chain.

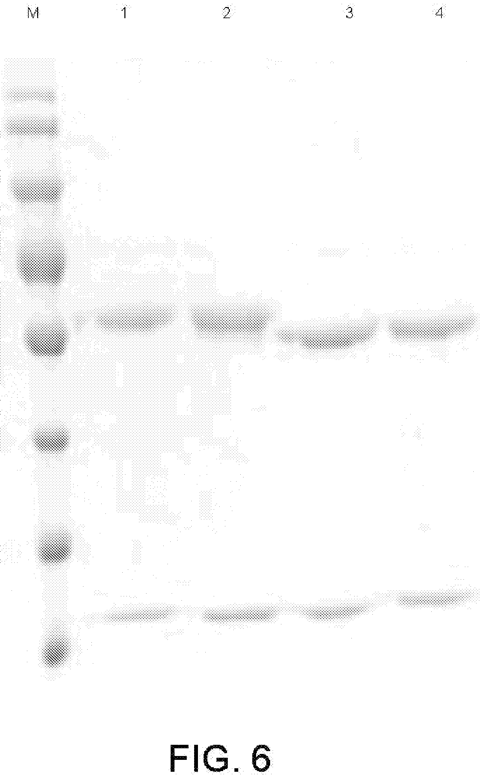

As illustrated in Table 7, the light and heavy chain expression vectors were co-transfected to HEK293 cells and were transiently expressed. After 3-4 days, supernatants were harvested. Four types of recombinant antibodies were purified using the use of Protein G affinity chromatography and the purity thereof is more than 95% (FIG. 6).

The chimeric-FMC63 is murine-human chimeric antibody (i.e., commonly referred to as the first generation of humanized antibody); wherein the variable regions are identical to antibodies of the murine donor while the constant region was replaced with human antibody constant region. Thus, the chimeric antibodies theoretically fully maintain biological activity of the antibodies of the murine donor. For the rest of humanized antibodies, various version of humanized heavy chains were adopted while the light chains are the same as the light chain of Chimeric FMC63 (See Table 7).

**TABLE 7**

| | Chimeric-FMC63 | hFMC63 | h1FMC63 | h2FMC63 |
|---|---|---|---|---|
| Heavy chain expression plasmid | pTSEG1 n -FMC63V H | pTSEG1n-h FMC63VH | pTSEG1n-h1 FMC 63VH | pTSEG1 n-h2 FMC 63VH |
| Light chain expression plasmid | pTSEK-F MC63VK | pTSEK-FM C63VK | pTSEK-FMC 63VK | pTSEK-FMC 63VK |

Activity comparison of heavy chains of the humanized antibody molecules was performed using Raji cells. Raji cell is a strain derived from human B lymphoma cell line. Raji cells were purchased from Chinese Academy of Medical Sciences, Institute of Basic Medical Cell Resource Center. The surface of Raji cells expresses antigen CD19, and anti-CD19 antibody can be used to analyze the binding capacity of recombinant humanized monoclonal antibodies. By flow cytometry (IFA), binding abilities of the four recombinant humanized antibodies were analyzed. The binding analysis were performed between four humanized FMC63 and antigen CD19 expressed on Raji cell surfaces. The results shows that the third version of humanized antibody (h2FMC63) binds to the antigen expressed on the surface of Raji cells. Further, the binding ability of h2FMC63 reaches 82% of chimeric FMV63. This indicates that humanized heavy chain h2FMC63VH remains the binding ability to human CD19 antigen as compared with the biding ability of chimeric FMV63 (See Table 8).

**TABLE 8**

| Humanized antibody | MFI (Geo Mean) | Relative affinity |
|---|---|---|
| Chimeric-FMC(FMC) | 404 | 100% |
| hFMC | 67 | 17% |
| h1FMC | 186 | 46% |
| h2FMC | 330 | 82% |

### Humanization of light chains of murine antibody FMC63

Humanized light chain of FMC63 were designed and generated by first building a light chain mutant library with storage capacity reaching 10⁸. The human-derived light chain replacement library (Fab library) was built using a dual-carrier phage display system and the humanized heavy chain gene (h2FMC63VH). The correct rate for the light chain library is above 90%.

The light chain mutant library was screened and various clones were identified. Recombinant CD19-His antigens were prepared. Using CD19-His antigens, the light chain mutant library were screened two rounds and enriched. More than 400 clones were identified using phage-ELISA, and more than 80% of these clones were positive. Among these clines, eight clones with the highest positive values were selected for further sequence analysis. The result shows that these 18 clones have different sequences. Among these 18 different clones, four clones were Lambda light chains, while the remaining 14 clones were kappa light chains.

The 18 clones were then prepared and purified to obtain phage-Fabs. After quantitation of the purified phage-Fabs, relative affinity analysis was performed on these phage-Fab using phage-ELISA. The result shows that three clones (L2D8, L2D12, L5F3) have the highest affinity values (Fig. 5). Sequences of these three light chains are provided in Table 9.

**TABLE 9**

| Name | Sequence | SEQ ID NO: |
|---|---|---|
| L2D8 | DIQMTQSPSSLSASVGDRVTITCRASQSVGSFLAWY QQKPGKAPKLLIYGASSRESGVPSRFSGSGSGTDFT LTISSLQPEDFATYYCQQTYHNPETFGQGTKVEIK | 34 |
| L2D12 | SYELTQPPSVSVAPGQTARITCGGNDLRAQYVHWY QQKPGQAPVLVMYDDSKRPSGIPERFSGSNSGNTA TLTISGTQAEDEADYYCQSWDRTSEPKVFGGGTKLT VL | 35 |
| L5F3 | SYELTQPPSVSVAPGQTARITCGGNNLGDNSARWY QQKPGQAPVLVIYGNSNRPSGIPERFSGSNSGNTAT LTISGTQAEDEADYYCQVTDTRSTSVVFGGGTKLTVL | 36 |

### Recombinant humanized murine antibody FMC63

Three light chain gene (L2D8, L2D12, L5F3) were selected and cloned into light chain expression vectors. Using HEK293 transient expression system, three humanized monoclonal antibodies were obtained. Three kinds of people humanized antibody heavy chain gene are h2FMC63VH (pTSEG1n-h2FMC63VH).

Preliminary analysis on antibody activity was performed using cell lines expressing human CD19 CD19-K562 (3 #, 20141224) and flow cytometry (FCM). The results are illustrated in Table 10.

**TABLE 10**

| antibody Sample | FMC63VH + FMC63VK | h2FMC63VH + L5F3 | h2FMC63VH +L2D12 | h2FMC63VH + L2D8 |
|---|---|---|---|---|
| MFI(Geo) | 47.2 | 147.3 | 55.5 | 125.3 |

The above data show that three new humanized antibody (L5F3, L2D12, L2D8) shows stronger binding abilities than murine antibody FMC63, wherein L5F3 and L2D8 shows significant improvement with respect to binding ability.

## Claims

1. A humanized antibody or fragment for binding to CD19 comprising
a heavy chain variable region (HVR) sequence comprising the amino acid sequence of SEQ ID NO: 27, and a light chain variable region (LVR) sequence comprising one of the amino acid sequences of SEQ ID NO: 34, SEQ ID NO: 35, or SEQ ID NO: 36.

2. The humanized antibody or fragment of claim 1, wherein the LVR sequence comprises the amino acid sequences of SEQ ID NO: 34.

3. The humanized antibody or fragment of claim 1, wherein the LVR sequence comprises the amino acid sequences of SEQ ID NO: 35.

4. The humanized antibody or fragment of claim 1, wherein the LVR sequence comprises the amino acid sequences of SEQ ID NO: 36.

5. The humanized antibody or fragment of any one of claims 1 to 4, wherein the HVR is joined to a human IgG chain constant region.

6. The humanized antibody or fragment of claim 5, wherein the human IgG is IgG1 or IgG3.

7. The humanized antibody fragment of any one of claims 1 to 4, wherein the fragment is a Fab, Fab', F(ab')2, Fv fragment; diabody; linear antibody; or single-chain antibody molecule.

8. The humanized antibody fragment of claim 7, wherein the fragment is a Fv fragment.

9. A composition comprising the humanized antibody or fragment of any one of claims 1 to 8 and a pharmaceutically acceptable carrier.

10. A humanized antibody or fragment according to any one of claims 1 to 8, or a composition according to claim 9, for use in therapy.

11. A humanized antibody or fragment according to any one of claims 1 to 8, or a composition according to claim 9, for use in the treatment of a CD19 positive tumor.

12. A humanized antibody or fragment according to any one of claims 1 to 8, or a composition according to claim 9, for use in the treatment of an autoimmune disease.

## Patentansprüche

1. Ein humanisierter Antikörper oder ein Fragment davon zur Bindung an CD19, umfassend
eine Sequenz der variablen Region der schweren Kette (HVR), umfassend die Aminosäuresequenz SEQ ID NO: 27, und eine Sequenz der variablen Region der leichten Kette (LVR), umfassend eine der Aminosäuresequenzen SEQ ID NO: 34, SEQ ID NO: 35 oder SEQ ID NO: 36.

2. Der humanisierte Antikörper oder das Fragment davon nach Anspruch 1, wobei die LVR-Sequenz die Aminosäuresequenzen SEQ ID NO: 34 umfasst.

3. Der humanisierte Antikörper oder das Fragment davon nach Anspruch 1, wobei die LVR-Sequenz die Aminosäuresequenzen SEQ ID NO: 35 umfasst.

4. Der humanisierte Antikörper oder das Fragment davon nach Anspruch 1, wobei die LVR-Sequenz die Aminosäuresequenzen SEQ ID NO: 36 umfasst.

5. Der humanisierte Antikörper oder das Fragment davon nach einem der Ansprüche 1 bis 4, wobei die HVR mit einer konstanten Region der Human-lgG-Kette verbunden ist.

6. Der humanisierte Antikörper oder das Fragment davon nach Anspruch 5, wobei das Human-IgG IgG1 oder IgG3 ist.

7. Das humanisierte Antikörperfragment nach einem der Ansprüche 1 bis 4, wobei das Fragment ein Fab-, Fab'-, F(ab')2-, Fv-Fragment, Diakörper, linearer Antikörper oder einkettiges Antikörpermolekül ist.

8. Das humanisierte Antikörperfragment nach Anspruch 7, wobei das Fragment ein Fv-Fragment ist.

9. Eine Zusammensetzung, die den humanisierten Antikörper oder das Fragment davon nach einem der Ansprüche 1 bis 8 und einen pharmazeutisch annehmbaren Träger umfasst.

10. Ein humanisierter Antikörper oder ein Fragment davon gemäß einem der Ansprüche 1 bis 8 oder eine Zusammensetzung gemäß Anspruch 9 zur Verwendung in der Therapie.

11. Ein humanisierter Antikörper oder ein Fragment davon gemäß einem der Ansprüche 1 bis 8 oder eine Zusammensetzung gemäß Anspruch 9 zur Verwendung bei der Behandlung eines CD19-positiven Tumors.

12. Ein humanisierter Antikörper oder ein Fragment davon gemäß einem der Ansprüche 1 bis 8 oder eine Zusammensetzung gemäß Anspruch 9 zur Verwendung bei der Behandlung einer Autoimmunerkrankung.

## Revendications

1. Anticorps humanisé ou fragment pour une liaison à CD19 comprenant
une séquence de région variable de chaîne lourde (HVR) comprenant la séquence d'acides aminés de SEQ ID NO: 27, et une séquence de région variable de chaîne légère (LVR) comprenant une des séquences d'acides aminés de SEQ ID NO: 34, SEQ ID NO: 35, ou SEQ ID NO: 36.

2. Anticorps humanisé ou fragment selon la revendication 1, dans lequel la séquence de LVR comprend la séquence d'acides aminés de SEQ ID NO: 34.

3. Anticorps humanisé ou fragment selon la revendication 1, dans lequel la séquence de LVR comprend la séquence d'acides aminés de SEQ ID NO: 35.

4. Anticorps humanisé ou fragment selon la revendication 1, dans lequel la séquence de LVR comprend la séquence d'acides aminés de SEQ ID NO: 36.

5. Anticorps humanisé ou fragment selon l'une quelconque des revendications 1 à 4, dans lequel la HVR est jointe à une région constante de chaîne d'IgG humaine.

6. Anticorps humanisé ou fragment selon la revendication 5, dans lequel l'IgG humaine est une IgG1 ou une IgG3.

7. Fragment d'anticorps humanisé selon l'une quelconque des revendications 1 à 4, où le fragment est un fragment Fab, Fab', F(ab')2, Fv ; un dianticorps ; un anticorps linéaire ; ou une molécule d'anticorps à chaîne unique.

8. Fragment d'anticorps humanisé selon la revendication 7, où le fragment est un fragment Fv.

9. Composition comprenant l'anticorps humanisé ou le fragment selon l'une quelconque des revendications 1 à 8 et un véhicule pharmaceutiquement acceptable.

10. Anticorps humanisé ou fragment selon l'une quelconque des revendications 1 à 8, ou composition selon la revendication 9, destiné(e) à être utilisé(e) dans une thérapie.

11. Anticorps humanisé ou fragment selon l'une quelconque des revendications 1 à 8, ou composition selon la revendication 9, destiné(e) à être utilisé(e) dans le traitement d'une tumeur positive pour CD19.

12. Anticorps humanisé ou fragment selon l'une quelconque des revendications 1 à 8, ou composition selon la revendication 9, destiné(e) à être utilisé(e) dans le traitement d'une maladie auto-immune.
